**Europäisches Patentamt**

**European Patent Office**

**Office européen des brevets**

(11) Veröffentlichungsnummer: **0 533 010 A2**

# EUROPÄISCHE PATENTANMELDUNG

(21) Anmeldenummer: **92115269.0**

(22) Anmeldetag: **07.09.92**

(51) Int. Cl.⁵: **C12N 15/52**, C12N 15/82, C12N 1/21, A01H 5/00, C12N 5/10, A01N 65/00

(30) Priorität: **18.09.91 DE 4130986**

(43) Veröffentlichungstag der Anmeldung:
**24.03.93 Patentblatt 93/12**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Kindl, Helmut, Prof. Dr.**
**Blaue Hofstadt 8**
**W-3550 Marburg(DE)**
Erfinder: **Hain, Rüdiger, Dr.**
**Talstrasse 53a**
**W-4018 Langenfeld(DE)**
Erfinder: **Reif, Hans-Jörg, Dr.**
**Gottesweg 165**
**W-5000 Köln 41(DE)**
Erfinder: **Stenzel, Klaus, Dr.**
**Seesener Strasse 17**
**W-4000 Düsseldorf 13(DE)**
Erfinder: **Thomzik, Jürgen, Dr.**
**Sauerbruchstrasse 13a**
**W-4018 Langenfeld(DE)**

(54) **Pinosylvinsynthase-Gene.**

(57) Die vorliegende Erfindung betrifft neue aus Pflanzen isolierte Gene für Pinosilvinsynthase und ihre Verwendung zur Transformation von Vektoren, Wirtsorganismen und Pflanzen sowie zur Erzeugung von Pflanzen, welche eine erhöhte Resistenz gegenüber Schädlingen aufweisen.

EP 0 533 010 A2

Die vorliegende Erfindung betrifft neue aus Pflanzen isolierte Gene für Pinosilvinsynthase und ihre Verwendung zur Transformation von Vektoren, Wirtsorganismen und Pflanzen sowie zur Erzeugung von Pflanzen, welche eine erhöhte Resistenz gegenüber Schädlingen aufweisen.

Als Pinosylvin wird das 3,5-Dihydroxy-stilben bezeichnet, welches in Pflanzen vorkommt und gegenüber Schädlingen, insbesondere Pilzen, Bakterien und Insekten toxisch wirkt und somit geeignet ist, diese Schädlinge abzuwehren. Die Fähigkeit der Synthese dieser Substanzen durch die Pflanzen wird als wichtiger Abwehrmechanismus angesehen. Leider haben nur wenige Nutzpflanzen die Fähigkeit Pinosylvin zu bilden, bzw. in einem Maße zu erzeugen, welches ihnen eine ausreichende Resistenz gegen Schädlinge verleiht.

Die Verwendung von Stilbensynthase-Genen zur Erzeugung von Pflanzen mit einer erhöhten Schädlingresistenz ist bereits aus der EP-A-0 309 862 bekannt. In dieser Veröffentlichung wird speziell ein Resveratrolsynthase-Gen aus Erdnußpflanzen (Arachis hypogea) beschrieben.

Es wurden nun neue Gene für Pinosylvinsynthase ("Pinosylvinsynthase-Gene") gefunden, welche in die Erbmasse (das Genom) von Pflanzen eingebaut werden können, die kein Pinosylvin oder nur unzureichend Pinosylvin erzeugen, wodurch eine erhöhte Resistenz dieser Pflanzen gegen Schädlinge hervorgerufen werden kann.

Überraschenderweise ergeben die neuen Pinosylvinsynthase-Gene wesentlich günstigere Schädlingsresistenzen in Pflanzen als das aus dem Stand der Technik vorbekannte Resveratrolsynthase-Gen aus Erdnuß.

Unter Pinosylvinsynthase-Genen soll jede Nukleinsäure (DNA) verstanden werden, die nach ihrer Transkription in RNA und Translation in Protein die Bildung eines Enzyms bewirkt, welches die Eigenschaften einer Pinosylvinsynthase besitzt, wobei diese Nukleinsäure aus ihrer natürlichen Umgebung isoliert oder in einen Vektor integriert ist oder in einer prokaryontischen oder eukaryontischen DNA als "fremde" DNA oder als "zusätzliche" DNA enthalten ist.

Unter Pinosylvinsynthase-Genen sollen auch solche Pinosylvinsynthase-Gene verstanden werden, die an ihrem Anfang und/oder Ende noch DNA-Sequenzen enthalten, die die Funktion der Gene nicht oder nicht wesentlich behindern. Diese auch als "Gen-Einheiten" bezeichneten DNA-Sequenzen entstehen, z.B. durch das Herausschneiden mit Restriktionsenzymen, da keine Schnittstellen für übliche Restriktionsenzyme exakt am Beginn und am Ende des Gens vorliegen. Die Pinosylvinsynthase-Gene bzw. die Gen-Einheiten können auch an ihren Enden solche DNA Sequenzen tragen, welche für ihre Handhabung jeweils angepaßt sind (z. B. "Linker").

Die Pinosylvinsynthase-Gene (bzw. die Gen-Einheiten) können in der Form vorliegen, wie sie im Genom von Pflanzen enthalten sind ("genomische" Form, einschließlich nicht Pinosylvinsynthase kodierender und/oder nicht regulatorisch wirkender Sequenzen (wie Introns)) oder in einer Form, welche der cDNA ("copy" DNA) entspricht, die über mRNA mit Hilfe von Reverse-Transkriptase/Polymerase erhältlich ist (und keine Introns mehr enthält). Die Pinosylvinsynthase-Gene können auch in teilweise oder vollständig synthetischer Form vorliegen. Unter synthetischen Genen werden auch solche verstanden, welche durch das neue Zusammenfügen von Teilen natürlicher Gene entstehen.

In den erfindungsgemäßen Pinosylvinsynthase-Genen (bzw. den Gen-Einheiten) können DNA-Abschnitte durch im wesentlichen gleichwirkende andere DNA-Abschnitte oder DNA's ersetzt sein.

Im vorliegenden Zusammenhang soll unter "fremder" DNA, solche DNA verstanden werden, welche in einem bestimmten prokaryontischen oder eukaryontischen Genom nicht natürlich vorkommt, sondern erst durch Eingriffe durch den Menschen in dieses Genom aufgenommen wird. "Zusätzliche" DNA soll solche DNA sein, welche in dem jeweiligen prokaryontischen oder eukaryontischen Genom zwar natürlich vorkommt, jedoch in zusätzlicher Menge durch Eingriffe durch den Menschen in dieses Genom aufgenommen wird. Die "fremde" DNA oder "zusätzliche" DNA kann je nach Bedarf und Art des vorliegenden Falles in einem oder mehreren Exemplaren eingebaut werden.

Pinosylvinsynthase, welche unter Mitwirkung der erfindungsgemäßen Pinosylvinsynthase-Gene (bzw. der Gen-Einheiten) in Pflanzen oder Pflanzenzellen gebildet wird, bedeutet jedes Enzym, welches wie Pinosylvinsynthase wirkt und in Pflanzen deren Resistenz gegenüber Schädlingen erhöht.

Die bevorzugten erfindungsgemäßen Pinosylvinsynthase-Gene sind dadurch gekennzeichnet, daß sie mit der im Plasmid pin 5-49 enthaltenen cDNA-Sequenz oder ihren Teilen bzw. mit der cDNA-Sequenz gemäß SEQ ID No: 1 oder ihren Teilen hybridisieren und für Pinosylvinsynthase codieren.

Erfindungsgemäß bevorzugte Pinosylvinsynthase-Gene sind die Pinosylvinsynthase-Gene, welche in Kiefer (Pinus sp.), besonders bevorzugt in Pinus sylvestris, vorkommen und daraus isoliert werden können.

Ganz besonders bevorzugt wird als erfindungsgemäßes Pinosylvinsynthase-Gen das Pinosylvinsynthase-Gen, dessen Teilsequenz in Form der cDNA auf dem Plasmid pin 5-49, (welches weiter unten näher beschrieben wird) vorliegt, sowie die im wesentlichen gleichwirkenden DNA-Sequenzen.

2

Die auf dem Plasmid enthaltene cDNA wurde aus Pinus sylvestris isoliert. Sie besteht aus einer ca. 1 300 Basenpaaren langen Sequenz. Eine Teilsequenz von 570 Basenpaaren geht aus dem Sequenzprotokoll SEQ ID No: 1 hervor.

Es wurde gefunden, daß die in Pflanzen (insbesondere Kiefer und besonders bevorzugt Pinus sylvestris) vorkommenden Pinosylvinsynthase-Gene über weite Bereiche eine DNA-Sequenzhomologie aufweisen. Die erfindungsgemäßen Pinosylvinsynthase-Gene können daher auf Grund der Sequenzhomologie mit Hilfe der auf dem Plasmid pin 5-49 enthaltenen cDNA oder ihrer Teile oder den Sequenzinformationen gemäß SEQ ID No:1 in üblicher Weise mit den bekannten Methoden der Molekularbiologie aus Pflanzen in einfacher Weise isoliert werden.

Als Pflanzen, aus denen erfindungsgemäße Pinosylvinsynthase-Gene isoliert werden können, kommen praktisch alle ein- oder zweikeimblättrige Pflanzen, vorzugsweise zweikeimblättrige Pflanzen in Frage, wobei beispielhaft und bevorzugt Kiefer (Pinus sp.) und besonders bevorzugt Pinus sylvestris genannt seien.

Wie bereits erwähnt, werden erfindungsgemäß Pinosylvinsynthase-Gene bzw. deren codierende Region bevorzugt, die mit der cDNA hybridisieren, welche auf dem Plasmid pin 5-49 liegt. Das Gen bzw. die codierende Region des Gens kann mit Hilfe der cDNA in üblicher Weise erhalten werden.

Der Escherichia coli Stamm E. coli pin 5-49 enthält das Plasmid pin 5-49. Dieser Stamm wurde bei der Deutschen Sammlung von Mikroorganismen (DSM), Mascheroder Weg 1b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt (Hinterlegungsdatum: 29. August 1991). Er erhielt die Hinterlegungsnummer DSM 6689.

Dieser Stamm sowie seine Mutanten sind ebenfalls Teil der vorliegenden Erfindung. Das in diesem Wirt hinterlegte Plasmid pin 5-49 kann in üblicher Weise durch die Vermehrung des Stammes und anschließende Isolierung des Plasmides leicht in den benötigten Mengen gewonnen werden.

Funktionell vollständige Gene, wie die erfindungsgemäßen Pinosylvinsynthase-Gene, bestehen aus einem regulatorisch wirkenden Teil (insbesondere Promotor) und dem Strukturgen, welches das Protein Pinosylvinsynthase kodiert.

Beide Genteile können unabhängig voneinander verwendet werden. So ist es möglich, dem regulativ wirkenden Teil eine (vom Pinosylvinsynthase-Gen abweichende) andere DNA-Sequenz nachzuschalten, welche nach dem Einbau in das Pflanzengenom exprimiert werden soll. Da nur relativ wenige isolierte Promotoren bekannt sind, welche ihre Wirkung in Pflanzen bzw. Pflanzenzellen entfalten können, stellen die Promotoren der Pinosylvinsynthase-Gene, welche ebenfalls Bestandteile der vorliegenden Erfindung sind, wertvolle Hilfsmittel bei der Erzeugung transformierter Pflanzen bzw. Pflanzenzellen dar.

Ebenso ist es möglich, den Pinosylvinsynthase-Struktur-Genen einen "fremden" regulatorisch wirkenden Teil vorzuschalten. Dies könnte vorteilhaft sein, wenn bei bestimmten Pflanzen nur bestimmte (z.B. pflanzeneigene) regulatorisch wirkende Gene ausreichend wirksam werden können. Die Pinosylvinsynthase-Struktur-Gene stellen somit wertvolle, selbständig einsetzbare Einheiten dar und sind, wie bereits dargelegt, ebenfalls Teil der vorliegenden Erfindung. Die erfindungsgemäßen Pinosylvinsynthase-Gene können nach den üblichen Methoden in die regulatorisch wirkenden Teile und die Struktur-Gene getrennt werden. Es ist auch möglich, Teile von verschiedenen natürlich vorkommenden Pinosylvinsynthase-Genen zu neuen funktionellen "synthetischen" Genen zu kombinieren. Bevorzugt werden die vollständigen natürlichen erfindungsgemäßen Pinosylvinsynthase-Gene (bzw. die Gen-Einheiten) verwendet.

Mit Hilfe der üblichen Methoden ist es möglich, die Pinosylvinsynthase-Gene (bzw. die Gen-Einheiten) oder ihre Teile ein oder mehrfach (z.B. Tandemanordnung), vorzugsweise einfach, in beliebige prokaryontische (vorzugsweise bakterielle) oder eukaryontische (vorzugsweise pflanzliche) DNA als "fremde" oder "zusätzliche" DNA einzubauen. So kann z.B. die proteincodierende DNA mit regulatorischen Sequenzen versehen werden und in Pflanzen eingebaut werden. Die so"modifizierte" rekombinante DNA, welche z.B. zur Transformation von Pflanzen bzw. Pflanzenzellen verwendet werden kann und nach der Transformation in Pflanzen bzw. Pflanzenzellen enthalten ist, ist Bestandteil der vorliegenden Erfindung.

Die Pinosylvinsynthase-Gene (bzw. die Gen-Einheiten) und/oder ihre Teile sowie die rekombinante DNA können als "fremde" oder "zusätzliche" DNA in Vektoren (insbesondere Plasmiden, Cosmiden oder Phagen), in transformierten Mikroorganismen (vorzugsweise Bakterien, insbesondere Gram-negativen Bakterien, wie E. coli) sowie in transformierten Pflanzenzellen und Pflanzen bzw. in deren DNA enthalten sein. Solche Vektoren, transformierte Mikroorganismen (die auch diese Vektoren enthalten können) sowie die transformierten Pflanzenzellen und Pflanzen und deren DNA stellen Bestandteile der vorliegenden Erfindung dar.

Wie bereits angedeutet, werden erfindungsgemäß die Pinosylvinsynthase-Gene (bzw. die Gen-Einheiten) ein-oder mehrfach (an gleichen oder verschiedenen Stellen des Genoms) in das natürliche pflanzliche Genom eingebaut, wobei verschiedene Gene auch mit einander kombiniert werden können, Bei Pflanzen, welche bereits über die Fähigkeit der Pinosylvinsynthase-Synthese verfügen, kann der Einbau eines oder mehrerer erfindungsgemäßer Pinosylvinsynthase-Gene zu einem erheblich verbesserten Resistenzverhalten führen, Bei Pflanzen, die keine Pinosylvinsynthase-Gene enthalten, wird durch den Einbau solcher Gene ebenfalls eine erhöhte Schädlingsresistenz erreicht, Gegebenenfalls werden nur die erfindungsgemäß Strukturgene verwendet, wobei ein evtl. aus der jeweiligen Pflanze isoliertes regulatorisches DNA Element vorgeschaltet wird,

Die erhöhte Resistenz der erfindungsgemäßen transformierten Pflanzenzellen und Pflanzen ist von Bedeutung für Landwirtschaft und Forsten, für den Zierpflanzenanbau, den Heilpflanzenanbau und die Pflanzenzucht, Auch bei der Kultivierung von Pflanzenzellen, z.B. zur Gewinnung von pharmazeutisch brauchbaren Stoffen, ist es von Vorteil, Pflanzenzellen verfügbar zu haben, welche gegen den Befall durch mikrobielle Schädlinge, insbesondere Pilze, erhöhte Resistenzen aufweisen.

Die vorliegende Erfindung betrifft somit auch ein Verfahren zur Herstellung transgener Pflanzenzellen (einschließlich Protoplasten) und Pflanzen (einschließlich Pflanzenteile und Samen) mit erhöhter Resistenz gegen Schädlinge, welches dadurch gekennzeichnet ist, daß man

(a) ein oder mehrere Pinosylvinsynthase-Gene (bzw. Gen-Einheiten) und/oder Teile der Pinosylvinsynthase-Gene (bzw. der Gen-Einheiten) und/oder erfindungsgemäße rekombinante DNA in den Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls vermehrt und gegebenenfalls

(c) von den so erhaltenen transgenen Pflanzen der Elterngeneration oder weiterer daraus gewonnener Generationen die gewünschten Pflanzenteile (einschließlich Samen) gewinnt.

Die Verfahrensschritte (a), (b) und (c) können nach bekannten Verfahren und Methoden in üblicher Weise durchgeführt werden.

Transgene Pflanzenzellen (einschließlich Protoplasten) und Pflanzen (einschließlich Pflanzenteile und Samen), welche ein oder mehrere Pinosylvinsynthase-Gene (bzw. Gen-Einheiten) und/oder Teile der Pinosylvinsynthase-Gene (bzw. der Gen- Einheiten) als "fremde" oder "zusätzliche" DNA enthalten sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung der Pinosylvinsynthase-Gene (bzw. der Gen-Einheiten) und/oder ihrer Teile und/oder der erfindungsgemäßen rekombinanten DNA und/oder der erfindungsgemäßen rekombinanten Vektoren und/oder der erfindungsgemäßen transformierten Mikroorganismen zur Transformation von Pflanzenzellen (einschließlich Protoplasten) und Pflanzen (einschließlich Pflanzenteilen und Samen), die

(b) Verwendung der erfindungsgemäßen transgenen Pflanzenzellen (einschließlich Protoplasten) und Pflanzen (einschließlich Pflanzenteilen und Samen) zur Erzeugung von Vermehrungsmaterial sowie zur Erzeugung neuer Pflanzen und deren Vermehrungsmaterial, die

(c) Verwendung der erfindungsgemäßen Pinosylvinsynthase-Gene (bzw. der Gen-Einheiten) und/oder ihrer Teile und/oder der erfindungsgemäßen rekombinanten DNA zur Bekämpfung von Schädlingen sowie die

d) Verwendung der auf dem Plasmid pin 5-49 enthaltenen cDNA oder ihrer Teile sowie der den Sequenzinformationen gemäß Sequenzprotokoll SEQ ID NO:1 entsprechenden DNA Sequenzen zur Isolierung von Pinosylvinsynthase-Genen oder deren Teilen aus Pflanzen sowie zur Bestimmung von Pinosylvinsynthase-Genen in Pflanzen sowie (allgemein) bei der Erzeugung von transgenen Pflanzenzellen (einschließlich Protoplasten) und Pflanzen (einschließlich Pflanzenteilen und Samen).

Eine Anzahl verschiedener Methoden steht zur Verfügung, die Pinosylvinsynthase-Gene bzw. die Gen-Einheiten oder ihre Teile als "fremde" oder "zusätzliche" DNA in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Das genetische Material, welches für Pinosylvinsynthase kodiert, wird zusammen mit regulatorischen DNA-Sequenzen in die T-DNA von geeigneten Ti-Plasmiden eingesetzt (z.B. Zambryski et al. 1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation von gereinigter DNA, die das gewünschte Gen enthält in Pflanzenprotoplasten (z.B. Hain et al., 1985; Krens et al., 1982; Paszkowski et al., 1984) in Gegenwart von Polykationen oder Calziumsalzen und Polyethylenglykol.

4

Die DNA-Aufnahme kann auch zusätzlich durch ein elektrisches Feld (Elektroporation) begünstigt werden (z.B. Fromm et al., 1986).

Die DNA kann in bekannter Weise auch über Pflanzenpollen eingeführt werden, indem Pollen mit physikalisch beschleinigten Partikeln "beschossen" werden, welche die DNA tragen (Vgl. EP-A 0 270 356).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens (gemäß der Methode aus EP-A 116 718) werden die Gene bzw. Geneinheiten, die komplementär zur cDNA aus pin 5-49 im Genom von Pinus vorliegen, in isolierter Form in einen geeigneten intermediaeren E.coli Vektor z.B. pGV700 oder pGV710, (vergl. EP-A-116 718) bzw. vorzugsweise Derivaten davon, die zusätzlich ein Reportergen wie z.B. nptII (Herrera-Estrella et al. 1983) oder hpt (Van den Elzen et al 1986) enthalten, kloniert.

Das so konstruierte Plasmid wird auf Agrobacterium tumefaciens, das z.B. pGV 3850 bzw. Derivate davon enthält (Zambryski et al. 1983) mit üblichen Methoden (z.B. Van Haute et al. 1983) übertragen. Alternativ dazu kann die Pinosylvinsynthase-Geneinheit in einem binaeren Vektor, z.B. pCV001 oder pCV002 (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die Pinosylvinsynthase-Gene bzw. Gen-Einheiten in einer auf Pflanzen transferierbaren Form enthält wird im weiteren zur Pflanzentransformation verwendet.

In einer weiteren bevorzugten Ausführungsform werden die isolierten Pinosylvinsynthase-Geneinheiten gegebenenfalls zusammen mit einem anderen Plasmid, das ein Reportergen für Pflanzenzellen, z.B. für Kanamycin-Resistenz (z.B. Herrera-Estrella et al. 1983) oder eine Hygromycin-Resistenz (van den Elzen, 1986) enthält, vorzugsweise pLGV neo 2103 (Hain et al. 1985), pMON 129 (Fraley R.T. et al., Proc. National Acad. Sci. USA 80, 4803 (1983)), pAK 1003, pAK 2004 (Velten J. et al., EMBO Journ. Vol. 3, 2723 (1984)) oder pGSST neo 3 (pGSST3) (EP-A-189 707), in üblicher Weise durch direkten Gentransfer auf Pflanzenprotoplasten übertragen (z.B. Hain et al 1985). Dabei können das bzw. die Plasmide in zirkulärer, vorzugsweise jedoch in linearer Form, vorliegen. Bei der Verwendung eines Plasmids mit Reportergen werden kanamycinresistente Protoplasten dann auf Expression von Pinosylvinsynthase überprüft. Im anderen Fall (ohne Reportergen) werden die resultierenden Kalli auf die Expression des oder der Pinosylvinsynthase-Gene geprüft (Screening mit üblichen Methoden).

Transformierte (transgene) Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycin-sulfat in vitro (Reiss et al. 1984; Schreier et al. 1985) oder durch die Expression der Nopalinsynthase (nach Aerts et al. 1983) oder Pinosylvinsynthase durch Northern-Blot-Analyse und Western Blot-Analyse nachgewiesen. Die Pinosylvinsynthase und die Stilbene können auch in bekannter Weise mit Hilfe spezifischer Antikörper in transformierten Pflanzen nachgewiesen werden. Pinosylvinsynthase kann auch durch Enzymaktivitätstest nachgewiesen werden (Gehlert et al., 1990).

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche die erfindungsgemäßen Pinosylvinsynthase-Gene (bzw. die Gen-Einheiten) enthalten und welche Bestandteile der vorliegenden Erfindung sind, zeigen eine erheblich höhere Resistenz gegen Schädlinge, insbesondere pflanzenpathogene Pilze.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen und Pflanzenzellen, welche zur Vermehrung der transformierten Pflanzen und Pflanzenzellen verwendet werden können und ist somit ebenfalls Teil der vorliegenden Erfindung.

Im vorliegenden Zusammenhang bedeutet der Ausdruck "im wesentlichen gleichwirkende DNA-Sequenzen", daß die Erfindung auch solche Modifikationen umfaßt, bei welchen die Funktion der Pinosylvinsynthase-Gene und ihrer Teile nicht derart beeinträchtigt ist, daß Pinosylvinsynthase nicht mehr gebildet wird oder der regulatorische Genteil nicht mehr wirksam wird. Entsprechende Modifikationen können durch den Ersatz, die Hinzufügung und/oder die Entfernung von DNA-Abschnitten, einzelner Kodons und/oder einzelner Nukleinsäuren erfolgen.

Bei den erfindungsgemäß verwendbaren Mikroorganismen bedeutet "Mutanten" solche modifizierten Mikroorganismen, welche noch die für die Ausführung der Erfindung wesentlichen Merkmale aufweisen, insbesondere das Plasmid pin 5-49 enthalten.

Zu den Pflanzen, welchen durch den Einbau (Transformation) der erfindungsgemäßen Pinosylvinsynthase-Gene (bzw. der Gen-Einheiten) Resistenz bzw. eine erhöhte Resistenz gegenüber den Schädlingen verliehen werden kann, gehören praktisch alle Pflanzen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernden Pflanzen, z.B. Getreide (insbsondere Weizen, Roggen, Gerste, Hafer, Hirse, Reis und Mais), Kartoffel, Leguminosen (wie Hülsenfrüchte und insbesondere Alfalfa, Sojabohnen), Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen wie Rauwolfia und Digitalis. Besonders bevorzugt seien Kartoffel, Tomaten und Leguminosen genannt. Vorzugsweise werden die erfindungsgemäßen Pinosylvinsynthase-Gene als "fremde" DNA in den Genom von Pflanzen eingebaut.

Als Schädlinge, gegen welche mit Hilfe der erfindungsgemäßen Pinosylvinsynthase-Gene Resistenzen, bzw. erhöhte Resistenzen erzielt werden können, seien tierische Schädlinge, wie Insekten, Milben und Nematoden sowie mikrobielle Schädlinge, wie phytopathogene Pilze, Bakterien und Viren genannt. Besonders hervorgehoben werden mikrobielle Schädlinge, insbesondere phytopathogene Pilze.

Zu den schädlichen Insekten gehören insbesondere Insekten der Ordnungen:
Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere: Tarsonemus spp., Panonychus spp. und Tetranychus spp.

Zu den schädlichen Nematoden gehören insbesondere: Pratylenchus spp., Heterodera spp. und Meloidogyne spp.

Zu den mikrobiellen Schädlingen gehören insbesondere die phytopathogenen Pilze:
Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Zu den Viruserkankungen gehören insbesondere Mosaik-, Verzwergungs- und Vergilbungsvirosen.

Beispielhaft aber nicht begrenzend seien einige Erreger von virösen, pilzlichen und bakteriellen Erkrankungen, die unter die oben aufgezählten Oberbegriffe fallen, genannt:
Barley Yellow Dwarf Virus (BYDV), Potato Virus Y (PVY), Cucumber Mosaic Virus (CMV), Watermelon Mosaic Virus (WMV), Tristeza-Virus, Tobacco Mosaic Virus (TMV), Tobacco Necrosis Virus (TNV), Beet necrotic Yellow Vein Virus (BNYVV), Rhizomania-Virus.

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea
(Konidienform: Drechslera, Syn: Helminthosporium);

Cochliobolus-Arten, wie beispielsweise Cochliobolus sativus
(Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielwseise Pseudocercosporella herpotrichoides. Weiterhin sei Helminthosporium carbonum aufgeführt.

Die vorliegende Erfindung soll anhand der folgenden beispielhaften Ausführungen näher erläutert werden:

## 1. Isolierung des Gens für Pinosylvinsynthase aus Pinus

Pflanzen und Zellkulturen aus Pinus (Pinus sylvestris) enthalten die Gene für Pinosylvinsynthase, welche die Bildung von Pinosylvinsynthase (Größe des Proteins 45 00 D; Reaktion mit spezifischem Antiserum) bewirken (Gehlert et al. 1990).

Bei der Isolierung der Pinosylvinsynthase-Gene wurden die bekannten Verfahren und Methoden der Molekularbiologie verwendet, wie sie beispielsweise in folgendem Handbuch detailliert beschrieben werden: Sambrook, J., Fritsch, E.F., Maniatis, T.: Molecular Cloning: A Laboratory Manual; Cold Spring Harbor Laboratory, Second Edition 1989.

Es wird zunächst eine "Gen-Bibliothek" für Pinus angelegt: Genomische DNA aus angereicherten Zellkernen (Bedbrook, J., Plant Molecular Biology Newsletter 2, 24, 1981) wird mit dem Restriktions-Enzym NdeII so geschnitten, daß DNA-Fragmente mit einer Durchschnittslänge von etwa 12 000 Nukleotidpaaren entstehen. Diese Fragmente werden in die BamHI-Stelle von Lambda-Phage EMBL4 kloniert (Frischauf et al., J. Mol. Biol. 170, 827-842, 1983), und die Phagen werden in E. coli vermehrt. Die Gesamtheit der Phagen-Population enthält, kloniert in Teilstücken, die gesamte genomische DNA der Pinuszellen, und damit auch die Gene für Pinosylvinsynthasen (Multigenfamilie).

Die Gene für Pinosylvinsynthase, ihre mRNA und die Pinosylvinsynthasen-cDNA enthalten jeweils gleiche Nucleinsäuresequenzen, da sie voneinander abgeleitet werden können (Gen→mRNA→cDNA). Dies bedeutet, daß die Gene für Pinosylvinsynthase durch spezifische Hybridisierung mit der jeweiligen Pinosylvinsynthase-cDNA bzw. mit spezifischen Oligonukleotiden identifizierbar sind. Gemäß diesem Verfahren wurden genomische Phagen-Klone für Pinosylvinsynthasen identifiziert und in Tabak übertragen mit dem Ergebnis, daß Pinosylvin in den heterologen Pflanzen erzeugt wurde. Die transgenen Pflanzen wiesen eine erhöhte Resistenz gegen Pflanzenpathogene auf. Die Phagen mit den Genen werden durch Hybridisierung identifiziert, dann isoliert und vermehrt. Die in diesen Phagen klonierte genomische DNA aus Pinus wird weiter durch Analyse mit verschiedenen Restriktionsenzymen kartiert, und die Position der Pinosylvinsynthase-Gene wird durch weitere Hybridisierungs-Experimente mit cDNA- Sequenzen bzw. synthetischen Oligonukleotiden festgelegt. Schließlich werden die Gen-Einheiten durch Verdau mit Restriktionsenzymen aus dem Phagen herausgeschnitten, im entsprechend geschnittenen Plasmid-Vektor pUC18 kloniert (Fa. Gibco-BRL GmbH, Eggenstein, Bundesrepublik Deutschland), und als rekombinante Plasmide vermehrt.

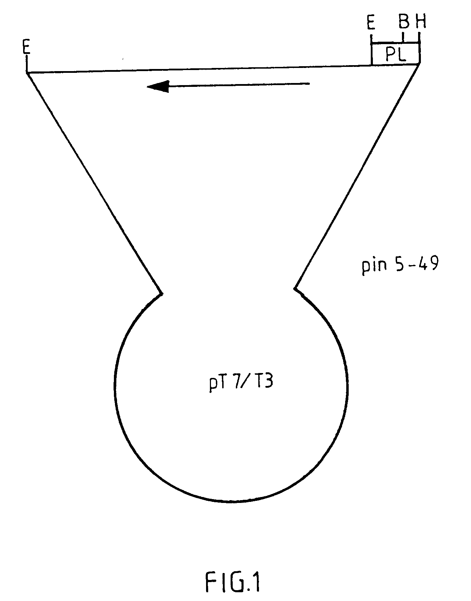

## 2. Beschreibung des Plasmids pin 5-49 (vgl. Fig. 1)

Das Plasmid besteht aus zwei Komponenten:

(i) cDNA (Teilsequenz) von Pinosylvinsynthase: Die cDNA, welche in das Plasmid pT7/T3 eingesetzt wurde, ist 1,3 kb lang und kann mit EcoRI aus dem Plasmid pin 5-49 herausgeschnitten werden.

(ii) Vektor-Plasmid: Die cDNA ist im Vektor pT7/T3 (Pharmacia LKB GmbH, Freiburg Bundesrepublik Deutschland) kloniert. Die Größe des Vektors ist 2800 Nukleotidpaare. Er trägt das Gen für Ampicillin-Resistenz, d.h. E. coli-Zellen mit diesem Plasmid wachsen in Nährmedien, die das Antibiotikum Ampicillin enthalten. Ori: Bezeichnung für Sequenzen, die für die Vermehrung des Plasmids in E. coli notwendig sind.

Das Plasmid pin 5-49 trägt ein Gen für Ampicillin-Resistenz und enthält als Pinosylvinsynthase-cDNA das oben beschriebene EcoRI-Fragment mit etwa 1,3 kb. Es kann in E. coli Zellen, welche pin 5-49 enthalten (E. coli pin 5-49), in üblicher Weise vermehrt werden.

Bevorzugtes Nährmedium für E. coli-Zellen (z.B. JA221, Nakamura, K., Inouye, M., EMBO J. 1, 771-775, 1982) welche pin 5-49 enthalten (E. coli pin 5-49):

```
Bacto-Pepton*      10 g

Hefeextrakt         5 g

NaCl                5 g

Agar               20 g

H₂O                 1 l

pH 7,5

Fermentation: 37°C, aerob
```

(* Bacto ist ein Warenzeichen der Fa. DIFCO Lab. Detroit, USA).

3. Transformation von Tabak

a) Kultur von Tabaksprossen und Isolierung von Tabakprotoplasten:

Nicotiana tabacum (Petit Havana SR1) wird als sterile Sproßkultur auf hormonfreiem LS Medium (Linsmaier und Skoog 1965) vermehrt. In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26°C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 m Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf $1-2 \times 10^5$/ml verdünnt. Die Protoplastenkonzentration wird in einer Zählkammer bestimmt.

b) Transformation von regenerierenden Tabakprotoplasten durch Kokultur mit Agrobacterium tumefaciens:

Es wird im folgenden die Methode von Marton et al. 1979 mit kleinen Veränderungen benutzt. Die Protoplasten werden wie beschrieben isoliert und in einer Dichte von $1-2 \times 10^5$/ml in K3 Medium (0,4 m Saccharose, 0,1 mg/l NAA, 0,2 ml in K3 Medium (0,4 m Saccharose, 0,1 mg/l NAA, 0,2 mg Kinetin) 2 Tage im Dunkeln und ein bis zwei Tage lang unter Schwachlicht (500 lux) bei 26°C inkubiert. Sobald die ersten Teilungen der Protoplasten auftreten, werden 30 $\mu$l einer Agrobakteriumsuspension in minimal A (Am) Medium (Dichte ca. $10^9$ Agrobakterien/ml) zu 3 ml regenerierenden Protoplasten gegeben. Die Kokulturdauer beträgt 3-4 Tage bei 20°C im Dunkeln. Danach werden die Tabakzellen in 12 ml Zentrifugenröhrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von $5 \times 10^4$/ml in K3 Medium (0,3 m Saccharose) mit 1 mg/l NAA (Naphthyl-1-essigsäure), 0,2 mg/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert, Die Selektion mit Kanamycin (100 mg/l Kanamycinsulfat (Sigma), 660 mg/g aktives Km) wird 2-3 Wochen nach der Kokultur in Agarose "bead type culture" (Shillito et al. 1983) gestartet. Kanamycinresistente Kolonien können 3-4 Wochen nach Beginn der Selektion vom Hintergrund zurückgebliebener Kolonien unterschieden werden.

c) Direkte Transformation von Tabakprotoplasten mit DNA. Calciumnitrat-PEG Transformation.

In einer Petrischale werden ca. $10^6$ Protoplasten in 180 $\mu$l K3 Medium mit 20 $\mu$l wäßriger DNA Lösung welche 0,5 $\mu$g/$\mu$l Plasmid, welches das genomische Pinosylvinsynthasegen trägt und 0,5 $\mu$g/$\mu$l pLGV neo 2103 (Hain et al. 1985) enthält, vorsichtig gemischt. Anschließend werden 200 $\mu$l Fusionslösung (0,1 m Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer kleinen Menge K3 Medium aufgenommen und wie im nächsten Abschnitt beschrieben kultiviert. Alternativ können die Protoplasten nach Hain et al. 1985 tranformiert werden.

Die Transformation kann auch ohne den Zusatz der 0,5 $\mu$g/$\mu$l pLGV neo 2103 durchgeführt werden. Da in diesem Fall kein Reportergen eingesetzt wird, werden die resultierenden Kalli auf das Vorhandensein der Pinosylvinsynthase-Gen-Einheit mit Hilfe einer Dot-Blot-Hybridisierung überprüft. Als Hybridisierung-Probe ist die cDNA Sequenz aus pin 5-49 verwendbar. Selbstverständlich können auch andere Nachweismethoden, wie Test mit Antikörpern oder Feststellung einer Pilz-Resistenz eingesetzt werden.

d) Kultur der mit DNA inkubierten Protoplasten und Selektion Kanamycin resistenter Kalli:

Für die im folgenden beschriebene Kultur und Selektion Kanamycin resistenter Kolonien wird eine modifizierte "Bead Type culture"-Technik (Shillito et al. 1983) verwendet. Eine Woche nach Behandlung der Protoplasten mit DNA (vgl. c) werden 3 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoklaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt.

Pro Woche wird das Austauschmedium (K3 + Km) um 0,05 m an Saccharose (ca. 60 mOsm) reduziert.

Schema der Selektion kanamycinresistenter Tabakkolonien nach DNA Transformation:

```
0,4 M  0,3 M  0,25 M  0,20 M  0,15M  0,10 M  Saccha

                                              rose im

                                              Flüssig

                                              medium

A   E S                                 K

────────────────────────────────────────────────────

      1      2      3      4      5      6  Wochen

                                              nach

DNA


Aufnahme

      (K3 Medium 1 mg NAA, 0,2 mg Kinetin)

A = DNA Aufnahme

E = Einbettung in Agarose

S = Selektion mit Kanamycin (100 mg/l Kanamycinsulfat)

K = Kanamycinresistente Kolonien können vom Hintergrund

      eindeutig unterschieden werden
```

e) Regeneration kanamycinrestistenter Pflanzen:

Sobald die kanamycinrestistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24°C im Kulturraum gehalten. Die andere Hälfte wird als Kalluskultur auf LS Medium mit 1 mg/l NAA, 0,2 mg/l Kinetin, 0,1 mg/l BAP und 100 mg/l Kanamydinsulfat propagiert. Wenn die regenerierten Sproße ca. 1 cm groß sind, werden sie abgeschnitten und auf 1/2 LS Medium (1 % Saccharose, 0,8 % Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

f) Tranformation von Blattscheiben durch Agrobacterium tumefaciens

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechen-der Agrobacterien (ca. $10^9$/ml) (vgl. b) in Am-Medium, siehe unten) für ca. 5 Minuten inkubiert. Die infizierten Blattstücke werden auf MS-Medium (siehe unten) ohne Hormone für 3-4 Tage bei ca. 24°C gehalten. Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 $\mu$g/ml Cefotaxim und 100 $\mu$g/ml Kanamycinsulfat (Sigma) gelegt. Nach zwei Wochen sollte das Medium erneuert werden. Transformierte Sproße werden nach weiteren 2-3 Wochen sichtbar. Die Regeneration von Sproßen sollte parallel auch ohne Selektionsdruck durchgeführt werden. Die regenerierten Sproße müssen dann durch biologische Tests z.B. auf Nopalinsynthase oder Pinosylvinsynthase Aktivität auf Transformation getestet weren. Auf diese Weise werden 1-10 % transformierte Sproße erhalten.

Biochemische Nachweismethode der Transformation

Nachweis von Nopalin in Pflanzengeweben:

Nopalin wird wie bei Otten und Schilperoort (1978) und Aerts et al. (1979) beschrieben, wie folgt, nachgewiesen. 50 mg Pflanzenmaterial (Kallus oder Blattstücke) werden über Nacht in LS Medium mit 0,1 M Arginin bei Raumtemperatur in einem Eppendorfgefäß inkubiert. Das Pflanzenmaterial wird danach auf saugfähigem Papier abgetupft, in einem frischen Eppendorfzentrifugengefäß mit einem Glasstab homogenisiert und 2 Min. in einer Eppendorfzentrifuge zentrifugiert. 2 $\mu$l des Überstandes werden auf ein für Elektrophorese geeignetes Papier (Whatman 3 MM Papier) (20 x 40 cm) punktförmig aufgetragen und getrocknet. Das Papier wird mit dem Laufmittel (5 % Ameisensäure, 15 % Essigsäure, 80 % $H_2O$, pH 1,8) getränkt und bei 400 V für 45 Minuten elektrophoretisiert. Nopalin läuft zur Kathode hin. Das Papier wird dann mit einem Luftstrom heiß getrocknet und durch Phenanthrenchinon-Färbemittel (gleiches Volumen 0,02 % Phenanthrenchinon in Ethanol und 10 % NaOH in 60 % Ethanol) in Laufrichtung gezogen. Das getrocknete Papier wird unter langwelligem UV-Licht betrachtet und fotografiert. Arginin und Argininderivate werden mit dem Reagenz gelb fluoreszierend gefärbt.

Neomycin-Phosphotransferase (NPT II) Enzymtest:

NPT II Aktivität in Pflanzengewebe wird durch in situ Phosphorylierung von Kanamycin, wie bei Reiß et al. (1984) beschrieben und von Schreier et al. (1985) modifiziert, wie folgt, nachgewiesen. 50 mg Pflanzengewebe werden in 50 $\mu$l Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 $\mu$l des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0,165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min, lang und einmal 30 Min. mit Reaktionspuffer gewaschen (67 mM Tris-Maleat, pH 7,1, 42 mM $MgCl_2$, 400 mM Ammoniumchlorid), Das Gel wird auf eine gleichgroße Glasplatte gelegt und mit 40 ml 1 %iger Agarose in Reaktionspuffer, der die Substrate Kanamycinsulfat (20 $\mu$g/ml) und 20-200 $\mu$Ci $^{32}P$ ATP (Amersham) enthält, überschichtet. Das Sandwichgel wird 30 Min. bei Zimmertempreratur inkubiert und dann wird ein Blatt Phosphozellulosepapier P81 (Whatman) auf die Agarose gelegt. Darüber werden vier Filtrierpapierlagen 3 MM, (Whatman) und einige Papierhandtücher gestapelt. Der Transfer von in situ phosphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7,5 bei 80°C gewaschen, getrocknet und für 1-12 h lang bei -70°C autoradiografiert (XAR5 Film Kodak),

4. Transformation von Solanum tuberosum (Kartoffel)

Die Transformation wurde genau nach dem in der EP-A-0 242 246, Seiten 14 bis 15 angegebenen Weise transformiert, wobei die Agrobakterien Ti-Plasmide enthalten, die Pinosylvinsynthase-Gene tragen.

Alle Prozentangaben in den obigen Beispielen beziehen sich auf Gewichtsprozente, wo nichts anderes angegeben wird.

In den gemäß den obigen Beispielen erhaltenen Pflanzenzellen und Pflanzen (Tabak) wurde die Anwesenheit der Pinosylvinsynthase-Gene durch Southern Blot Analyse bestätigt. Die Expression der Pinosylvinsynthase-Gene wurde durch Northern Blot Analyse, Pinosylvinsynthase und Pinosylvin mit Hilfe von spezifischen Antikörpern nachgewiesen. Transformierte und nicht-transformierte Pflanzen (zum Vergleich) wurden mit einer Sporensuspension von Botrytis cinera besprüht und nach 1 Woche der Pilzbefall bonitiert. Die transformierten Pflanzen zeigten (gegenüber den nicht transformierten Vergleichspflanzen) eine erhöhte Resistenz gegen Pilzbefall.

Hybridisierung mit der cDNA-Sequenz von Plasmid pin 5-49 bzw. der cDNA-Sequenz gemäß SEQ ID No: 1

Wie oben ausgeführt sind die bevorzugten erfindungsgemäßen Pinosylvinsynthase-Gene dadurch gekennzeichnet, daß sie bei der im Plasmid pin 5-49 enthaltenen cDNA-Sequenz oder ihren Teilen bzw. mit der cDNA-Sequenz gemäß SEQ ID No: 1 oder ihren Teilen hybridisieren und für Pinosylvinsynthase codieren. Die Hybridisierung kann allgemein auch für die Bestimmung und Isolierung von Pinosylvinsynthase-Genen z.B. in Planzen oder Pflanzenteilen eingesetzt werden.

Vorzugsweise Phagen-Klone die Pinosylvinsynthase-Gene enthalten, können durch Hybridisierung mit pin 5-49 (bzw. SEQ ID No: 1) unter niedrig stringenten Bedingungen identifiziert werden. Es wird eine Subpopulation von Klonen erhalten, die nachfolgend als Pinosylvinsynthase-Gene-Klone identifiziert werden können, z.B. durch direkten Gen-Transfer in Pflanzen (Hain et al, 1985 und 1990) und anschließende Analyse des transgenen Pflanzengewebes auf enzymatische Pinosylvinsynthase-Aktivität oder auf Pinosylvin.

Beispielhaft wurden Pinosylvinsnthase-Gen-Klone mit Hilfe des cDNA-Klones 5-49 (bzw. SEQ ID Nr: 1) als Sonde identifiziert, wobei Standard Hybridisierungsbedingungen eingesetzt werden. Die Hybridisierung erfolgte 12 Stunden bei 68°C in Standard-Puffer enthaltend 2 SSC. Gewaschen wurde bei 74°C in 2 SSC und 0,1 % SDS (2 mal 30 Min.) mit einer nachfolgenden Waschung in 0,2 SSC und 0,1 % SDC (10 Min.). Die Phagen-Klon-DNA wurde mit einem pflanzenselektiven Marker (Kanamycin-Resistenz) in Tabak-Protoplasten co-übertragen und die Pinosylvinsynthase in Tabak nachgewiesen.

Im folgenden werden einige der bei der Transformation von Pflanzen bzw. Pflanzenzellen eingesetzte Medien beschrieben:

Am-Medium

| | |
|---|---|
| 3,5 g | $K_2HPO_4$ |
| 1,5 g | $KH_2PO_4$ |
| 0,5 g | $Na_3$ Citrat |
| 0,1 g | $MgSO_4$ x $7H_2O$ |
| 1 g | $(NH_4)_2SO_4$ |
| 2 g | Glukose |
| ad 1 l | |

Medium für sterile Snroßkultur von Tabak

Macro-elemente 1/2 der Konzentration der MS Salze
Micro-elemente 1/2 der Konzentration der MS Salze

| Fe-EDTA | Murashige und Skoog (MS) | |
|---|---|---|
| Myo-Inosit | | 100 mg/l |
| Sucrose | | 10 mg/l |
| Agar | | 8 g/l |
| Vitamine | Ca-panthotenat | 1 mg/l |
| | Biotin | 10 mg/l |
| | Nicotinsäure | 1 mg/l |
| | Pyridoxin | 1 mg/l |
| | Thiamin | 1 mg/l |
| pH 5,7 vor dem Autoklavieren | | |

K3-Medium

Zur Kultur von Nicotiana tabacum petit Havana SR1, Nicotiana tabacum Wisconsin 38, und Nicotiana plumbaginifolia Protoplasten (Nagy und Maliga, 1976)

| Macro-elemente | $NH_4NO_3$ | 250 mg/l |
|---|---|---|
| | $KNO_3$ | 2500 mg/l |
| | $CaCl_2 \cdot 2H_2O$ | 900 mg/l |
| | $MgSO_4.7H_2O$ | 250 mg/l |
| | $NaH_2PO_4.1H_2O$ | 150 mg/l |
| | $(NH_4)_2SO_4$ | 134 mg/l |
| | $CaHPO_4.1H_2O$ | 50 mg/l |
| Micro-elemente | $H_3BO_3$ | 3 mg/l |
| | $MnSO_4.1H_2O$ | 10 mg/l |
| | $ZnSO_4.4H_2O$ | 2 mg/l |
| | KI | 0,75 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit Sucrose | | 100 mg/l |
| | | 137 g/l |
| | | ( = 0,4 M) |
| Xylose Vitamine | | 250 mg/l |
| | Nicotinsäure | 1 mg/l |
| | Pyridoxin | 1 mg/l |
| | Thiamin | 10 mg/l |
| Hormone | NAA | 1,0 mg/l |
| | Kinetin | 0,2 mg/l |
| pH 5,6 Filter sterilisieren | | |

Linsmaier und Skoog Medium (Linsmaier und Skoog 1965)

Zur Kultur von regenerierenden Protoplasten und für Gewebekultur von Tabaktumoren und Kallus. Linsemaier und Skoog (LS) Medium ist Murashige und Skoog Medium (Murashige und Skoog, 1962) mit den folgenden Modifikationen:
- Thiamin wird in höherer Konzentration eingewogen 0,4 mg/l anstatt 0,1 mg/l;

- Glycin, Pyridoxin und Nicotinsäure fehlen.

| Macro-elemente | $NH_4NO_3$ | 1650 mg/l |
| | $KNO_3$ | 1900 mg/l |
| | $CaCl_2.2H_2O$ | 440 mg/l |
| | $MgSO_4.7H_2O$ | 370 mg/l |
| | $KH_2PO_4$ | 170 mg/l |
| Micro-elemente | $H_3BO_3$ | 6,2 mg/l |
| | $MnSO_4.1H_2O$ | 22,3 mg/l |
| | $ZnSO_4.4H_2O$ | 8,6 mg/l |
| | KI | 0,83 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Saccharose | | 30 g/l |
| Agar | | 8 g/l |
| Vitamine | Thiamin | 0,4 mg/l |
| Hormone: | NAA | 1 mg/l |
| | Kinetin | 0,2 mg/l |
| pH 5,7 vor dem Autoklavieren | | |

Zur Transformation von Pflanzen bzw. Pflanzenzellen kann die folgende Literatur angeführt weren:

Aerts M, Jacobs M, Hernalsteens JP, Van Montagu M, Schell J (1983) Induction and in vitro culture of Arabidopsis thaliana crown gall tumours. Plant Sci Lett. 17: 43-50

Fromm ME, Taylor LP, Walbot V (1986) Stable transformation of maize after gene transfer by electroporation. Nature 319: 791-793

Gehlert, R., Schöppner, A., and Kindl, H. (1990) Synthase from Seedlings of Pinus sylvestris: Purification and Induction in Response to Fungal Infection. Molecular Plant-Microbe Interactions. Vol 3, No 6, p.p. 444-449

Hain, R., Stabel, P., Czernilofsky, A.Pp., Steinbiß, H.H., Herrera-Estrella, L., Schell, J. (1985) Uptake, integration, expression and genetic transmission of a selectable chimeric gene by plant protoplasts. Molec Gen Genet 199: 161-168

Herrera-Estrella L., De Block M., Messens E., Hernalsteens JP., van Montagu M., Schell J. (1983) EMBO J. 2: 987-995.

Horsch RB, Fry JE, Hoffmann NL, Eichholtz D, Rogers SG, Fraley RT (1985) A simple and general method for transferring genes into plants. Science 277: 1229-1231

Krens FH, Molendijk L, Wullems GJ, Schilperoort RA (1982) in vitro transformation of plant protoplasts with Ti-plasmid DNA. Nature 296: 72-74

Koncz C, Schell J (1986) The promotor of $T_L$-DNA gene 5 controls the tissue-specific expression of chimaeric genes carried by a novel type of Agrobacterium binary vector. Mol. Gen. Genet. (1986) 204: 338-396

Linsmaier DM, Skoog F (1965) Organic growth factor requirements of tobacco tissue cultures. Physiol Plant 18: 100-127

Marton L, Wullems GJ, Molendijk L, Schilperoort PR (1979) In vitro transformation of cultured cells from Nicotiana tabacum by Agrobacterium tumefaciens. Nature 277: 1229-131

Murashige, T. and Skoog F. (1962) A revised medium for rapid growth and bioassay with tobacco tissue culture. Physiol. Plant. 15, 47

Nagy JI, Maliga P (1976) Callus induction and plant regeneration from mesophyll protoplasts of Nicotiana sylvestris. Z Pflanzenphysiol 78: 453-455

Otten LABM, Schilperoort RA (1978) A rapid microscale method for the detection of Lysopin and Nopalin dehydrogenase activities. Biochim biophys acta 527: 497-500

Paszkowski J, Shillito RD, Saul M, Mandak V, Hohn T, Hohn B, Potrykus I (1984) Direct gene transfer to plants. EMBO J 3: 2717-2722

Shillito RD, Paszkowski J. Potrykus I (1983) Agarose plating and Bead type culture technique enable and stimulate development of protoplast-derived colonies in an number of plant species. PI Cell Rep 2: 244-247

Van den Elzen PJM, Townsend J, Lee KY, Bedbrook JR (1985) Achimaeric resistance gene as a selectable marker in plant cells. Plant Mol. Biol. 5, 299-302.

Van Haute E, Joos H, Maes M, Warren G, Van Montagu M, Schell J (1983) Intergenic transfer and exchange recombination of restriction fragments cloned in pBR322: a novel strategy for the reversed genetics of Ti plasmids of /Agrobacterium tumefaciens. EMBO J 2: 411-418

Velten J, Velten L, Hain R, Schell J (1984) Isolation of a dual plant promotor fragment from the Ti Plasmid of Agrobacterium tumefaciens. EMBO J 12: 2723-2730

Wullems GJ, Molendijk L, Ooms G, Schilperoort RA (1981) Differential expression of crown gall tumor markers in transformants obtained after in vitro Agrobacterium tumefaciens - induced transformation of cell wall regenerating protoplasts derived from Nicotiana tabacum. Proc Natl Acad Sci 78: 4344-4348

Zambryski P, Joos H, Genetello C, van Montagu M, Schell J (1983) Ti-plasmid vector for the introduction of DNA into plant cells without altering their normal regeneration capacity, EMBO J 12: 2143-2150.

Reiss B, Sprengel R, Will H and Schaller H (1984) A new sensitive method for qualitative and quantitative assay of neomycin phosphotransferase in crude cell tracts, GENE 1081: 211-217

Schreier P, Seftor E, Schell J and Bohnert H (1985) The use of nuclear-encoded sequences to direct the light-regulated synthesis and transport of a foreign protein into plant chloroplasts, EMBO J Vol. 4, No. 1: 25-32

Weiterhin können die folgenden veröffentlichten Patentanmeldungen aufgeführt werden:

EP-A 116 718
EP-A 159 418
EP-A 120 515
EP-A-120 516
EP-A-172 112
EP-A-140 556
EP-A-174 166
EP-A-122 791
EP-A-126 546
EP-A-164 597
EP-A-175 966
WO 84/02913
WO 84/02919
WO 84/02920
WO 83/01176


Erläuterungen zu Fig. 1


Fig. 1                    stellt das Plasmid pin 5-49 dar. Die Pinosylvinsynthase-cDNA liegt auf dem ca. 1,3 kb großen EcoRI Fragment.

In Fig. 1 bedeuten:

E : EcoRI
B : Bam HI
H : Hind III
PI: Polylinker aus dem Plasmid pT7/T3

SEQ ID NO:1

ART DER SEQUENZ: Nucleotid mit entsprechendem Peptid

SEQUENZLÄNGE: 570 Basenpaare

STRANGEFORM: Einzelstrang

TOPOLOGIE: linear

ART DES MOLEKÜLS: cDNA

URSPRÜNGLICHE HERKUNFT:

ORGANISMUS: Pinus sylvestris

UNMITTELBARE EXPERIMENTELLE HERKUNFT: Pinus sylvestris

NAME DER ZELLINIE:

MERKMALE: von 1 bis 570 reife Peptidsequenz

EIGENSCHAFTEN: Teilsequenz für Pinosylvinsynthase aus Pinus sylvestris

```
  1  aagaatcccgatgtgtgcgcgttcgtggaggtgccatcgttggacgcacggcaggccatg  60
  1  K   N   P   D   V   C   A   F   V   E   V   P   S   L   D   A   R   Q   A   M    20

 61  ttggctatggaggtgccccggctggcaaaagaggccgctgaaaaggccattcaggagtgg  120
 21  L   A   M   E   V   P   R   L   A   K   E   A   A   E   K   A   I   Q   E   W    40

121  gggcagtccaagtctgggatcactcatctcatattttgcagcacaacgactccggatcta  180
 41  G   Q   S   K   S   G   I   T   H   L   I   F   C   S   T   T   T   P   D   L    60

181  cctggagcagactttgaggtagccaagttgctggggctgcacccgagtgtgaagagagtg  240
 61  P   G   A   D   F   E   V   A   K   L   L   G   L   H   P   S   V   K   R   V    80

241  ggcgtgttccaacatggctgcttcgccggaggcaccgttcttcgaatggcgaaagacctt  300
 81  G   V   F   Q   H   G   C   F   A   G   G   T   V   L   R   M   A   K   D   L    100

301  gccgaaaacaatcgaggagctcgggtgctggtcatctgtagtgaaaccaccgccgttacc  360
101  A   E   N   N   R   G   A   R   V   L   V   I   C   S   E   T   T   A   V   T    120

361  tttcgtggaccctccgagactcacctggacagcctggtggggcaagctctgtttggcgac  420
121  F   R   G   P   S   E   T   H   L   D   S   L   V   G   Q   A   L   F   G   D    140

421  ggtgcttctgccctcatcgtgggagctgatcccatccctcaagtggagaaggcctgtttc  480
141  G   A   S   A   L   I   V   G   A   D   P   I   P   Q   V   E   K   A   C   F    160

481  gaaatcgtttggacagcccagacagttgttcccaacagcgagggagccatcggtgggaag  540
161  E   I   V   W   T   A   Q   T   V   V   P   N   S   E   G   A   I   G   G   K    180

541  gtgagagaggtcgggctgaccttccaactc  570
181  V   R   E   V   G   L   T   F   Q   L    190
```

## Patentansprüche

1. Pinosylvinsynthase-Gene.

2. Pinosylvinsynthase-Gene, welche dadurch gekennzeichnet sind, daß sie mit der im Plasmid pin 5-49 enthaltenen Pinosylvinsynthase-cDNA Sequenz oder ihren Teilen bzw. mit der cDNA-Sequenz gemäß SEQ ID NO:1 oder ihren Teilen hybridisieren und für Pinosylvinsynthase codieren.

3. Pinosylvinsynthase-Gene gemäß den Ansprüchen 1 und 2, erhältlich aus dikotyledonen Pflanzen.

**4.** Pinosylvinsynthase-Gene gemäß den Ansprüchen 1 bis 3, erhältlich aus Pinus.

**5.** Regulatorisch wirkender Teil der Pinosylvinsynthase-Gene gemäß den Ansprüchen 1 bis 4.

**6.** Struktur-Gene der Pinosylvinsynthase-Gene gemäß den Ansprüchen 1 bis 4.

**7.** Rekombinante prokaryontische oder eukaryontische DNA, welche ein oder mehrere Pinosylvinsynthase-Gene oder ihre Teile gemäß den Ansprüchen 1 bis 6 als "fremde" DNA oder als "zusätzliche" DNA enthält.

**8.** Rekombinante DNA gemäß Anspruch 8, welche in Pflanzenzellen (einschließlich Protoplasten) oder Pflanzen (einschließlich Pflanzenteilen und Samen) enthalten ist.

**9.** Vektoren, welche ein oder mehrere Pinosylvinsynthase-Gene oder ihre Teile gemäß den Ansprüchen 1 bis 8 und/oder rekombinante DNA gemäß Anspruch 7 enthalten.

**10.** Vektor-Plasmid pin 5-49.

**11.** Transformierte Mikroorganismen, welche ein oder mehrere Pinosylvinsynthase-Gene oder ihre Teile gemäß den Ansprüchen 1 bis 6 und/oder rekombinante DNA gemäß Anspruch 7 oder Vektoren gemäß den Ansprüchen 9 und 10 enthalten.

**12.** Escherichia coli Stamm E. coli pin 5-49 sowie seine Mutanten.

**13.** Verwendung der Pinosylvinsynthase-Gene und/oder ihrer Teile und/oder der rekombinanten DNA und/oder der Vektoren und/oder der transformierten Mikroorganismen gemäß den Ansprüchen 1 bis 12 zur Transformation von Pflanzenzellen (einschließlich Protoplasten) und Pflanzen (einschließlich Pflanzenteilen und Samen).

**14.** Transgene Pflanzenzellen (einschließlich Protoplasten) und Pflanzen (einschließlich Pflanzenteilen und Samen), welche ein oder mehrere Pinosylvinsynthase-Gene und/oder deren Teile und/oder die rekombinante DNA gemäß den Ansprüchen 1 bis 7 als "fremde" oder "zusätzliche" DNA enthalten.

**15.** Verfahren zur Herstellung transgener Pflanzenzellen (einschließlich Protoplasten) und Pflanzen (einschließlich Pflanzenteile und Samen) mit erhöhter Resistenz gegen Schädlinge, dadurch gekennzeichnet, daß man
(a) ein oder mehrere Pinosylvinsynthase-Gene oder ihre Teile und/oder die rekombinante DNA gemäß den Ansprüchen 1 bis 7 in den Genom von Pflanzenzellen (einschließlich Protoplasten) einsetzt und gegebenenfalls
(b) aus den transgenen Pflanzenzellen (einschließlich Protoplasten) vollständige transformierte Pflanzen regeneriert und gegebenenfalls vermehrt und gegebenenfalls
(c) von den so erhaltenen transgenen Pflanzen der Elterngeneration oder weiterer daraus gewonnener Generationen die gewünschten Pflanzenteile (einschließlich Samen) gewinnt.

**16.** Verwendung der transgenen Pflanzenzellen (einschließlich Protoplasten) und Pflanzen (einschließlich Pflanzenteilen und Samen) gemäß Anspruch 14 zur Erzeugung von Vermehrungsmaterial sowie zur Erzeugung neuer Pflanzen, die die Pinosylvinsynthase-Gene oder ihre Teile gemäß den Ansprüchen 1 bis 6 oder die rekombinante DNA gemäß Anspruch 7 enthalten und deren Vermehrungsmaterial.

**17.** Vermehrungsmaterial, erhältlich durch die Vermehrung der transgenen Pflanzenzellen und Pflanzen gemäß Anspruch 14.

**18.** Verwendung von DNA-Sequenzen, welche ganz oder teilweise der cDNA entsprechen, die auf dem Plasmid pin 5-49 enthalten ist, bzw. in SEQ ID NO:1 aufgeführt ist, zur Isolierung von Pinosylvinsynthase-Genen aus Pflanzen sowie bei der Erzeugung von transgenen Pflanzenzellen (einschließlich Protoplasten) und Pflanzen (einschließlich Pflanzenteilen und Samen).

FIG.1